(19) Europäisches Patentamt
European Patent Office
Office européen des brevets

(11) EP 2 673 011 B2

(12) NEUE EUROPÄISCHE PATENTSCHRIFT
Nach dem Einspruchsverfahren

(45) Veröffentlichungstag und Bekanntmachung des Hinweises auf die Entscheidung über den Einspruch:
**16.01.2019 Patentblatt 2019/03**

(45) Hinweis auf die Patenterteilung:
**08.04.2015 Patentblatt 2015/15**

(21) Anmeldenummer: **12702048.5**

(22) Anmeldetag: **07.02.2012**

(51) Int Cl.:
**A61L 15/60** (2006.01)     **C08L 33/10** (2006.01)
**A61F 13/53** (2006.01)

(86) Internationale Anmeldenummer:
**PCT/EP2012/052022**

(87) Internationale Veröffentlichungsnummer:
**WO 2012/107432 (16.08.2012 Gazette 2012/33)**

(54) **VERFAHREN ZUR HERSTELLUNG WASSERABSORBIERENDER POLYMERPARTIKEL MIT HOHER ANQUELLGESCHWINDIGKEIT**

PROCEDURE FOR PREPARING WATER ABSORBING POLYMER PARTICLES HAVING HIGH FREE SWELL RATE

PROCEDE POUR LA PRÉPARATION DES PARTICULES POLYMÈRES ABSORBANT DE L'EAU AYANT UNE HAUTE VITESSE DE GONFLEMENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.02.2011 EP 11153534**

(43) Veröffentlichungstag der Anmeldung:
**18.12.2013 Patentblatt 2013/51**

(73) Patentinhaber: **BASF SE**
**67056 Ludwigshafen am Rhein (DE)**

(72) Erfinder:
• **DANIEL, Thomas**
**67165 Waldsee (DE)**
• **BRAIG, Volker**
**69469 Weinheim-Lützelsachsen (DE)**
• **KARIM, Asif**
**68159 Mannheim (DE)**

(74) Vertreter: **BASF IP Association**
**BASF SE**
**G-FLP-C006**
**67056 Ludwigshafen (DE)**

(56) Entgegenhaltungen:
| US-A- 5 672 633 | US-A- 5 981 070 |
| US-A1- 2005 031 850 | US-A1- 2005 137 546 |
| US-A1- 2008 032 888 | US-A1- 2009 008 604 |
| US-A1- 2010 062 252 | US-A1- 2010 100 066 |

EP 2 673 011 B2

**Beschreibung**

Die vorliegende Erfindung betrifft ein Verfahren zur Herstellung wasserabsorbierender Polymerpartikel mit hoher Anquellgeschwindigkeit, umfassend die Schritte Polymerisation, Trocknung, Mahlung, Klassierung und thermische Oberflächennachvernetzung, Nachbefeuchtung und erneute Trocknung.

Wasserabsorbierende Polymerpartikel werden zur Herstellung von Windeln, Tampons, Damenbinden und anderen Hygieneartikeln, aber auch als wasserzurückhaltende Mittel im landwirtschaftlichen Gartenbau verwendet. Die wasserabsorbierenden Polymerpartikel werden oft auch als "absorbent resin", "superabsorbents ", "superabsorbent polymer", "absorbent polymer", "absorbent gelling material", "hydrophilic polymer", "Hydrogele" oder "Superabsorber" bezeichnet.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Die Eigenschaften der wasserabsorbierenden Polymerpartikel können beispielsweise über die verwendete Vernetzermenge eingestellt werden. Mit steigender Vernetzermenge sinkt die Zentrifugenretentionskapazität (CRC) und die Absorption unter einem Druck von 21,0 $g/cm^2$ (AUL0.3psi) durchläuft ein Maximum.

Zur Verbesserung der Anwendungseigenschaften, wie beispielsweise Flüssigkeitsweiterleitung (SFC) und Absorption unter einem Druck von 49.2 $g/cm^2$ (AUL0.7psi), werden wasserabsorbierende Polymerpartikel im allgemeinen oberflächennachvernetzt. Dadurch steigt der Vernetzungsgrad der Partikeloberfläche, wodurch die Absorption unter einem Druck von 49,2 $g/cm^2$ (AUL0.7ps i) und die Zentrifugenretentionskapazität (CRC) zumindest teilweise entkoppelt werden können. Diese Oberflächennachvernetzung kann in wässriger Gelphase durchgeführt werden. Vorzugsweise werden aber getrocknete, gemahlene und abgesiebte Polymerpartikel (Grundpolymer) an der Oberfläche mit einem Oberflächennachvernetzer beschichtet, thermisch oberflächennachvernetzt und getrocknet. Dazu geeignete Vernetzer sind Verbindungen, die mit mindestens zwei Carboxylatgruppen der wasserabsorbierenden Polymerpartikel kovalente Bindungen bilden können.

Die Nachbefeuchtung oberflächennachvernetzer Polymerpartikel wird beispielsweise in EP 0 480 031 A1, EP 0 780 424 A1, WO 01/025290 A1, WO 2004/037900 A1 und WO 2006/109844 A1 beschrieben.

EP 0 780 424 A1 lehrt, dass der Restepoxidgehalt nach der Oberflächennachvernetzung mit Epoxiden durch Zusatz eines Nukleophils wie Wasser gesenkt werden kann.

WO 01/025290 A1 beschreibt, dass durch Nachbefeuchtung die mechanische Stabilität der wasserabsorbierenden Polymerpartikel erhöht werden kann.

WO 2004/037900 A1 offenbart ein zweistufiges Mischverfahren zur Nachbefeuchtung.

EP 0 480 031 A1 und WO 2006/109844 A1 lehren die Verwendung von Wasser zur Agglomeration von wasserabsorbierenden Partikeln.

Aufgabe der vorliegenden Erfindung war die Bereitstellung eines Verfahrens zur Herstellung wasserabsorbierender Polymerpartikel mit verbesserter Anquellgeschwindigkeit.

Gelöst wurde die Aufgabe durch ein Verfahren zur Herstellung wasserabsorbierender Polymerpartikel durch Polymerisation einer Monomerlösung oder-suspension, enthaltend

a) mindestens ein ethylenisch ungesättigtes, säuregruppentragendes Monomer, das zumindest teilweise neutralisiert sein kann,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,

wobei das Polymergel getrocknet, gemahlen und klassiert wird, wobei die Polymerpartikel auf eine Partikelgröße im Bereich von m bis n $\mu$m klassiert werden, m eine Zahl im Bereich von 50 bis 300 und n eine Zahl im Bereich von 400 bis 1.200 bedeutet, die klassierten Polymerpartikel mit

f) mindestens einem Oberflächennachvernetzer und
g) optional mindestens einem polyvalenten Metallkation

beschichtet und thermisch oberflächennachvernetzt werden, dadurch gekennzeichnet, dass der Feuchtegehalt der wasserabsorbierenden Polymerpartikel nach der thermischen Oberflächennachvernetzung durch In-Kontakt-bringen mit Wasser in gasförmiger Form um 1 bis 150 Gew.-% erhöht wird und die wasserabsorbierenden Polymerpartikel anschließend getrocknet werden.

Die Klassierung wird mittels geeigneter Siebe mit den entsprechenden Maschenweiten durchgeführt, wobei m im Bereich von vorzugsweise 80 bis 250, besonders bevorzugt von 100 bis 200, ganz besonders bevorzugt von 110 bis 150, und m im Bereich von vorzugsweise 500 bis 1.000, besonders bevorzugt von 600 bis 900, ganz besonders bevorzugt von 700 bis 850, liegt.

Der Feuchtegehalt wird nach der thermischen Oberflächennachvernetzung um vorzugsweise 2,5 bis 100 Gew.-%, besonders bevorzugt 5 bis 50 Gew.-%, ganz besonders 10 bis 25 Gew.-%, erhöht (Nachbefeuchtung). Die wasserabsorbierenden Polymerpartikel werden mit Wasser in gasförmiger Form in Kontakt gebracht, beispielsweise durch Durchlüftung mit feuchten Gasen (Luft, Stickstoff, usw.).

Die Produkttemperatur beträgt während der Erhöhung des Wassergehalts beispielsweise 0 bis 140°C, vorzugsweise 20 bis 120°C, besonders bevorzugt 50 bis 100°C, ganz besonders bevorzugt 60 bis 90°C.

Die Verweilzeit zwischen Erhöhung des Wassergehalts und anschließender Trocknung ist unkritisch und beträgt beispielsweise weniger als 10 Tage, vorzugsweise weniger als 5 Tage, bevorzugt weniger als einen Tag, besonders bevorzugt weniger als 6 Stunden, ganz besonders bevorzugt weniger als 2 Stunden.

Die anschließende Trocknung kann statisch oder dynamisch durchgeführt werden, d.h. die wasserabsorbierenden Polymerpartikel werden dabei bewegt, beispielsweise gerührt, oder nicht. Vorzugsweise wird dynamisch getrocknet. Der Druck bei der Trocknung ist ebenfalls unkritisch und entspricht beispielsweise dem Umgebungsdruck oder weniger (Unterdruck). Es ist aber auch möglich die wasserabsorbierenden Polymerpartikel zur Trocknung mit einem troknn Gas (Luft, Stickstoff, usw.) zu durchlüften.

In einer bevorzugten Ausführungsform der vorliegenden Erfindung wird bei der erfindungsgemäßen Trocknung der gewünschte Feuchtegehalt für das Endprodukt eingestellt.

Anschließend werden die wasserabsorbierenden Polymerpartikel bei Temperaturen von vorzugsweise weniger als 150°C, besonders bevorzugt weniger als 130°C, ganz besonders bevorzugt weniger als 110°, bis zu einem Feuchtegehalt von vorzugsweise weniger als 10 Gew.-%, besonders bevorzugt weniger als 7 Gew.-%, ganz besonders bevorzugt weniger als 5 Gew.-%, getrocknet.

Der vorliegenden Erfindung liegt die Erkenntnis zugrunde, dass die Anquellgeschwindigkeit (FSR) wasserabsorbierender Polymerpartikel erhöht werden kann, indem die oberflächennachvernetzten Polymerpartikel gequollen und wieder getrocknet werden. Durch die Quellung bilden sich Risse in der höher vernetzten Schale. Diese Risse sind möglicherweise für die Erhöhung der Anquellgeschwindigkeit (FSR) ursächlich. Allerdings dürfen die wasserabsorbierenden Polymerpartikel nicht zu stark quellen, da ansonsten die Polymerpartikel untereinander verkleben. Weiterhin werden gemäß der vorliegenden Erfindung die zu kleinen Polymerpartikel vor der Oberflächennachvernetzung abgetrennt, so dass sich eine Agglomeration erübrigt.

Die für das erfindungsgemäße Verfahren einsetzbaren oberflächennachvernetzten Polymerpartikel weisen daher typischerweise eine hohe Flüssigkeitsweiterleitung (SFC) und einen hohen Vortex auf, beispielsweise eine Flüssigkeitsweiterleitung (SFC) von vorzugsweise mindestens $100 \times 10^{-7}$ cm$^3$s/g, besonders bevorzugt von mindestens $120 \times 10^{-7}$ cm$^3$s/g, ganz besonders bevorzugt von 130 bis $250 \times 10^{-7}$ cm$^3$s/g, und einen Vortex von vorzugsweise mindestens 60 s, besonders bevorzugt mindestens 80 s, ganz besonders bevorzugt von 100 bis 500 s.

Die für das erfindungsgemäße Verfahren einsetzbaren oberflächennachvernetzten Polymerpartikel weisen eine Zentrifugenretentionskapazität (CRC) von typischerweise mindestens 15 g/g, vorzugsweise mindestens 20 g/g, bevorzugt mindestens 22 g/g, besonders bevorzugt mindestens 24 g/g, ganz besonders bevorzugt 26 bis 60 g/g, auf. Die Zentrifugenretentionskapazität (CRC) wird gemäß der von der EDANA empfohlenen Testmethode Nr. WSP 241.2-05 "Fluid Retention Capacity in Saline, After Centrifugation" bestimmt.

Die für das erfindungsgemäße Verfahren einsetzbaren oberflächennachvernetzten Polymerpartikel weisen eine Absorption unter einem Druck von 49,2 g/cm$^2$ von typischerweise mindestens 15 g/g, vorzugsweise mindestens 20 g/g, bevorzugt mindestens 22 g/g, besonders bevorzugt mindestens 24 g/g, ganz besonders bevorzugt 26 bis 35 g/g, auf. Die Absorption unter einem Druck von 49,2 g/cm$^2$ wird analog der von der EDANA empfohlenen Testmethode Nr. WSP 242.2-05 "Absorption Under Pressure, Gravimetric Determination" bestimmt, wobei statt eines Drucks von 21,0 g/cm$^2$ ein Druck von 49,2 g/cm$^2$ eingestellt wird.

Im Folgenden wird die Herstellung der wasserabsorbierenden Polymerpartikel näher erläutert:

Die wasserabsorbierenden Polymerpartikel werden durch Polymerisation einer Monomerlösung oder-suspension hergestellt und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AM PS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraalloxyethan, Methylenbismethacrylamid, 15-fach ethoxiliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-flach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, ganz besonders bevorzugt 0,3 bis 0,6 Gew.-%, jeweils bezogen auf Monomer a). Mit steigendem Vernetzergehalt sinkt die Zentrifugenretentionskapazität (CRC) und die Absorption unter einem Druck von 21,0 g/cm$^2$ durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise Dinatrium-2-hydroxy-2-sulfonatoazetat oder ein Gemisch aus Dinatrium-2-hydroxy-2-sulfinatoazetat, Dinatrium-2-hydroxy-2-sulfonatoazetat und Natriumbisulfit eingesetzt. Derartige Gemische sind als Brüggolite® FF6 und Brüggolite® FF7 (Brüggemann Chemicals; Heilbronn; Deutschland) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssigem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Die Monomerlösung oder -suspension wird polymerisiert. Geeignete Reaktoren sind beispielsweise Knetreaktoren oder Bandreaktoren. Im Kneter wird das bei der Polymerisation einer wässrigen Monomerlösung oder-suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Verfahrensschritt zerkleinert werden muss, beispielsweise in einem Extruder oder Kneter.

Zur Verbesserung der Trocknungseigenschaften kann das mittels eines Kneters erhaltene zerkleinerte Polymergel zusätzlich extrudiert werden.

Es ist aber auch möglich eine wässrige Monomerlösung zu vertropfen und die erzeugten Tropfen in einem erwärmten Trägergasstrom zu polymerisieren. Hierbei können die Verfahrensschritte Polymerisation und Trocknung zusammengefasst werden, wie in WO 2008/040715 A2 und WO 2008/052971 A1 beschrieben.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 95 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Das erhaltene Polymergel wird getrocknet. Die Trockner unterliegen keiner Beschränkung. Die Trocknung des Polymergels wird aber vorzugsweise mit einem Bandtrockner durchgeführt bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA empfohlenen Testmethode Nr. WSP 230.2-05 "Mass Loss Upon Heating" bestimmt wird. Bei einer zu hohen Restfeuchte weist das getrocknete Polymergel eine zu niedrige Glasübergangstemperatur $T_g$ auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung vorzugsweise von 25 und 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein Schaufeltrockner verwendet werden.

Das getrocknete Polymergel wird gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise mindestens 200 μm, besonders bevorzugt von 250 bis 600 μm, ganz besonders von 300 bis 500 μm. Die mittlere Partikelgröße der Produktfraktion kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von mindestens 150 $\mu$m beträgt vorzugsweise mindestens 90 Gew.-%, besonders bevorzugt mindesten 95 Gew.-%, ganz besonders bevorzugt mindestens 98 Gew.-%.

Polymerpartikel mit zu niedriger Partikelgröße senken die Flüssigkeitsweiterleitung (SFC). Daher sollte der Anteil zu kleiner Polymerpartikel ("fines") niedrig sein.

Zu kleine Polymerpartikel werden daher üblicherweise abgetrennt und in das Verfahren rückgeführt. Dies geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt.

Werden die zu kleinen Polymerpartikel sehr früh zugesetzt, beispielsweise bereits zur Monomerlösung, so wird dadurch die Zentrifugenretentionskapazität (CRC) der erhaltenen wasserabsorbierenden Polymerpartikel gesenkt. Dies kann aber beispielsweise durch Anpassung der Einsatzmenge an Vernetzer b) kompensiert werden.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 $\mu$m, beträgt vorzugsweise mindestens 90 Gew.-%, besonders bevorzugt mindesten 95 Gew.-%, ganz besonders bevorzugt mindestens 98 Gew.-%.

Der Anteil an Partikeln mit einer Partikelgröße von höchstens 600 $\mu$m, beträgt vorzugsweise mindestens 90 Gew.-%, besonders bevorzugt mindesten 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Die Polymerpartikel werden zur Verbesserung der Eigenschaften thermisch oberflächennachvernetzt. Geeignete Oberflächennachvernetzer f) sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder ß-Hydroxyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidon und dessen Derivate, wie 2-Hydroxyethyl-2-oxazolidon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidone, in DE 102 04 937 A1 zyklische Harnstoffe, in DE 103 34 584 A1 bizyklische Amidacetale, in EP 1 199 327 A2 Oxetane und zyklische Harnstoffe und in WO 2003/031482 A1 Morpholin-2,3-dion und dessen Derivate als geeignete Oberflächennachvernetzer f) beschrieben.

Bevorzugte Oberflächennachvernetzer f) sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer f) sind 2-Hydroxyethyloxazolidin-2-on, Oxazolidin-2-on und 1,3-Propandiol.

Weiterhin können auch Oberflächennachvernetzer f) eingesetzt werden, die zusätzliche polymerisierbare ethylenisch ungesättigte Gruppen enthalten, wie in DE 37 13 601 A1 beschrieben

Die Menge an Oberflächennachvernetzer f) beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

In einer bevorzugten Ausführungsform der vorliegenden Erfindung werden vor, während oder nach der Oberflächennachvernetzung zusätzlich zu den Oberflächennachvernetzern polyvalente Metallkationen g) auf die Partikeloberfläche aufgebracht.

Die im erfindungsgemäßen Verfahren einsetzbaren polyvalenten Metallkationen g) sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat und Lactat, möglich. Aluminiumsulfat und Aluminiumlaktat sind bevorzugt.

Die Einsatzmenge an polyvalentem Metallkation g) beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf die Polymerpartikel.

Die Oberflächennachvernetzung wird üblicherweise so durchgeführt, dass eine Lösung des Oberflächennachvernetzers f) auf die getrockneten Polymerpartikel aufgesprüht wird. Im Anschluss an das Aufsprühen werden die mit Oberflächennachvernetzer f) beschichteten Polymerpartikel thermisch getrocknet, wobei die Oberflächennachvernetzungsreaktion sowohl vor als auch während der Trocknung stattfinden kann.

Das Aufsprühen einer Lösung des Oberflächennachvernetzers f) wird vorzugsweise in Mischern mit bewegten Mischwerkzeugen, wie Schneckenmischer, Scheibenmischer und Schaufelmischer, durchgeführt werden. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; USA) und Schugi Flexomix® (Hosokawa Micron BV; Doetinchem; Niederlande). Es ist aber auch möglich die Oberflächennachvernetzerlösung in einem Wirbelbett aufzusprühen.

Die Oberflächennachvernetzer f) werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers f) in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die thermische Oberflächennachvernetzung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; Deutschland), Hosokawa Bepex® Disc Dryer (Hosokawa Micron GmbH; Leingarten; Deutschland) und Nara Paddle Dryer (NARA Machinery Europe; Frechen; Deutschland). Überdies können auch Wirbelschichttrockner eingesetzt werden.

Die thermische Oberflächennachvernetzung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Oberflächennachvernetzungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten. Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nach der erfindungsgemäßen Trocknung erneut nachbefeuchtet werden.

Die Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die wasserabsorbierenden Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert.

Geeignete Beschichtungen zur Verbesserung der Anquellgeschwindigkeit sowie der Flüssigkeitsweiterleitung (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20.

Ein weiterer Gegenstand der vorliegenden Erfindung sind die gemäß dem erfindungsgemäßen Verfahren erhältlichen wasserabsorbierenden Polymerpartikel.

Ein weiterer Gegenstand der vorliegenden Erfindung sind nichtagglomerierte wasserabsorbierende Polymerpartikel, enthaltend

i) mindestens ein polymerisiertes ethylenisch ungesättigtes, säuregruppentragendes Monomer a), das zumindest teilweise neutralisiert sein kann,

ii) mindestens einen polymerisierten Vernetzer b),

iii) optional ein oder mehrere mit den unter a) genannten Monomeren copolymerisierte ethylenisch ungesättigte Monomere d) und

iv) optional ein oder mehrere wasserlösliche Polymere e),

v) mindestens einen umgesetzten Oberflächennachvernetzer f) und

vi) optional mindestens ein polyvalentes Metallkation g),

wobei die wasserabsorbierenden Polymerpartikel einen Feuchtegehalt von weniger als 5 Gew.-% und bei 20.000-facher Vergrößerung sichtbare Risse in der Partikeloberfläche aufweisen.

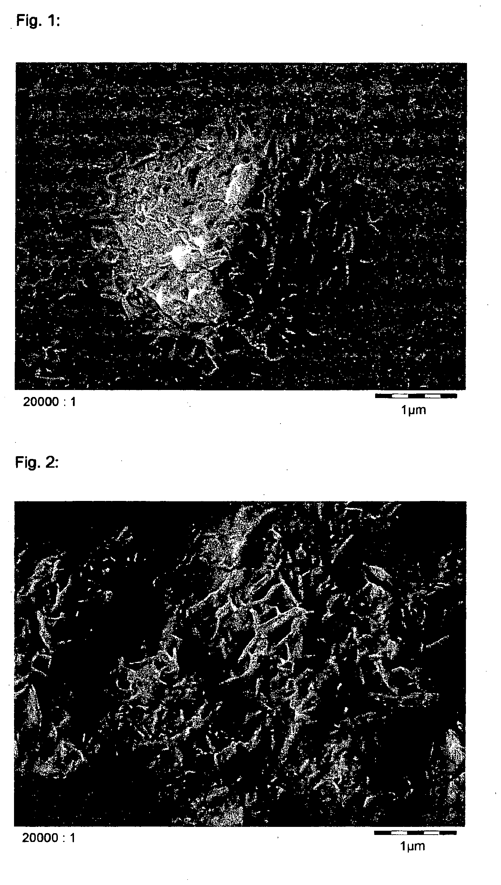

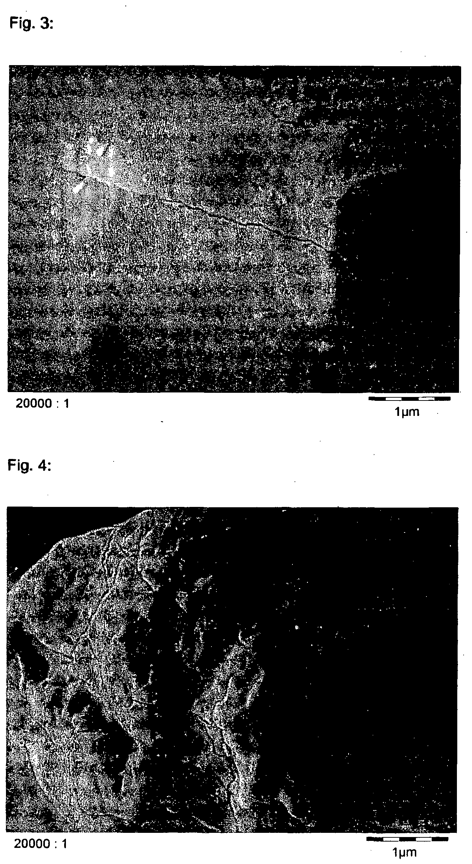

Die Figur 1 zeigt die Oberfläche eines Polymerpartikels ohne Risse und die Figuren 2 bis 4 zeigen Oberflächen wasserabsorbierender Polymerpartikel mit erfindungsgemäßen Rissen.

Das polymerisierte Monomer i) ist vorzugsweise Acrylsäure.

Die erfindungsgemäßen wasserabsorbierenden Polymerpartikel weisen typischerweise eine hohe Flüssigkeitsweiterleitung (SFC) und einen niedrigen Vortex auf, beispielsweise eine Flüssigkeitsweiterleitung (SFC) von vorzugsweise mindestens $100 \times 10^{-7}$ cm$^3$s/g, besonders bevorzugt von mindestens $120 \times 10^{-7}$ cm$^3$s/g, ganz besonders bevorzugt von 130 bis $250 \times 10^{-7}$ cm$^3$s/g, und einen Vortex von vorzugsweise weniger als 100 s, besonders bevorzugt von weniger als 80 s, ganz besonders bevorzugt von 20 bis 60 s.

Die erfindungsgemäßen wasserabsorbierenden Polymerpartikel weisen eine Zentrifugenretentionskapazität (CRC) von typischerweise mindestens 15 g/g, vorzugsweise mindestens 20 g/g, bevorzugt mindestens 22 g/g, besonders bevorzugt mindestens 24 g/g, ganz besonders bevorzugt 26 bis 60 g/g, auf. Die Zentrifugenretentionskapazität (CRC) wird gemäß der von der E-DANA empfohlenen Testmethode Nr. WSP 241.2-05 "Fluid Retention Capacity in Saline, After Centrifugation" bestimmt.

Die erfindungsgemäßen wasserabsorbierenden Polymerpartikel weisen eine Absorption unter einem Druck von 49,2 g/cm$^2$ von typischerweise mindestens 15 g/g, vorzugsweise mindestens 20 g/g, bevorzugt mindestens 22 g/g, besonders bevorzugt mindestens 24 g/g, ganz besonders bevorzugt 26 bis 35 g/g, auf. Die Absorption unter einem Druck von 49,2 g/cm$^2$ wird analog der von der EDANA empfohlenen Testmethode Nr. WSP 242.2-05 "Absorption Under Pressure, Gravimetric Determination" bestimmt, wobei statt eines Drucks von 21,0 g/cm$^2$ ein Druck von 49,2 g/cm$^2$ eingestellt wird.

Ein weiterer Gegenstand der vorliegenden Erfindung sind Hygieneartikel, enthaltend erfindungsgemäße wasserabsorbierende Polymerpartikel, insbesondere Hygieneartikel zur Damenhygiene, Hygieneartikel für leichte und schwere Inkontinenz oder Kleintierstreu.

Die Hygieneartikel enthalten üblicherweise eine wasserundurchlässige Rückseite, eine wasserdurchlässige Oberseite und dazwischen einen absorbierenden Kern aus den erfindungsgemäßen wasserabsorbierenden Polymerpartikeln und Fasern, vorzugsweise Cellulose. Der Anteil der erfindungsgemäßen wasserabsorbierenden Polymerpartikel im absorbierenden Kern beträgt vorzugsweise 20 bis 100 Gew.-%, bevorzugt 50 bis 100 Gew.-%.

Die wasserabsorbierenden Polymerpartikel werden mittels der nachfolgend beschriebenen Testmethoden geprüft.

Die mit "WSP" bezeichneten Standard-Testmethoden werden beschrieben in: "Standard Test Methods for the Nonwovens Industry", Ausgabe 2005, gemeinsam herausgegeben von den "Worldwide Strategic Partners" EDANA (Avenue Eugene Plasky 157, 1030 Brussels, Belgium, www.edana.org) und INDA (1100 Crescent Green, Cary, NC 27518, U.S.A., www.inda.org). Diese Veröffentlichung ist sowohl von EDANA als auch von INDA erhältlich.

Methoden:

Die Messungen sollten, wenn nicht anders angegeben, bei einer Umgebungstemperatur von 23 $\pm$ 2 °C und einer relativen Luftfeuchte von 50 $\pm$ 10 % durchgeführt werden. Die wasserabsorbierenden Polymerpartikel werden vor der Messung gut durchmischt.

pH-Wert

Der pH-Wert der wasserabsorbierenden Polymerpartikel wird gemäß der von der EDANA empfohlenen Testmethode Nr. WSP 200.2-02 "pH of Polyacrylate (PA) Powders" bestimmt.

Feuchtegehalt

Der Feuchtegehalt der wasserabsorbierenden Polymerpartikel wird gemäß der von der EDANA empfohlenen Testmethode Nr. WSP 230.2-02 "Mass Loss Upon Heating" bestimmt.

Zentrifugenretentionskapazität (Centrifuge Retention Capacity)

Die Zentrifugenretentionskapazität (CRC) wird gemäß der von der EDANA empfohlenen Testmethode Nr. WSP 241.2-05 "Fluid Retention Capacity in Saline, After Centrifugation" bestimmt.

Absorption unter einem Druck von 49,2 g/cm$^2$ (Absorption under Load)

Die Absorption unter einem Druck von 49,2 g/cm$^2$ (AUL0.7psi) wird analog der von der EDANA empfohlenen Testmethode Nr. WSP 242.2-05 "Absorption Under Pressure, Gravimetric Determination" bestimmt, wobei statt eines Drucks von 21,0 g/cm$^2$ (AUL0.3psi) ein Druck von 49,2 g/cm$^2$ (AUL0.7psi) eingestellt wird.

Extrahierbare

Der Gehalt an extrahierbaren Bestandteilen der wasserabsorbierenden Polymerpartikel wird gemäß der von der EDANA empfohlenen Testmethode Nr. WSP 270.2-05 "Extractable" bestimmt.

Anquellgeschwindigkeit (Free Swell Rate)

Zur Bestimmung der Anquellgeschwindigkeit (FSR) werden 1,00 g (= W1) der wasserabsorbierenden Polymerpartikel in ein 25 ml Becherglas eingewogen und gleichmäßig auf dessen Boden verteilt. Dann werden 20 ml einer 0,9 gew.-%igen Kochsalzlösung mittels eines Dispensers in ein zweites Becherglas dosiert und der Inhalt dieses Glases wird dem ersten zügig hinzugefügt und eine Stoppuhr gestartet. Sobald der letzte Tropfen Salzlösung absorbiert wird, was man am Verschwinden der Reflexion auf der Flüssigkeitsoberfläche erkennt, wird die Stoppuhr angehalten. Die genaue Flüssigkeitsmenge, die aus dem zweiten Becherglas ausgegossen und durch das Polymer im ersten Becherglas absorbiert wurde, wird durch Rückwägung des zweiten Becherglases genau bestimmt (=W2). Die für die Absorption benötigte Zeitspanne, die mit der Stoppuhr gemessen wurde, wird als t bezeichnet. Das Verschwinden des letzten Flüssigkeitstropfens auf der Oberfläche wird als Zeitpunkt t bestimmt.

Daraus errechnet sich die Anquellgeschwindigkeit (FSR) wie folgt:

$$FSR \ [g/g \ s] = W2/(W1 \ x \ t)$$

Wenn der Feuchtegehalt der wasserabsorbierenden Polymerpartikel jedoch mehr als 3 Gew.-% beträgt, so ist das Gewicht W1 um diesen Feuchtegehalt zu korrigieren.

Vortex

In ein 100 ml-Becherglas, welches ein Magnetrührstäbchen der Größe 30 mm x 6 mm enthält, werden 50,0 ml ± 1,0 ml einer 0,9 Gew.-%igen wässrigen Kochsalzlösung gegeben. Mit Hilfe eines Magnetrührers wird die Kochsalzlösung bei 600 Upm gerührt. Es werden dann möglichst schnell 2,000 g ± 0,010 g wasserabsorbierende Polymerpartikel zugegeben, und die Zeit gemessen, die vergeht, bis die Rührtraube durch die Absorption der Kochsalzlösung durch die wasserabsorbierenden Polymerpartikel verschwindet. Dabei kann der ganze Inhalt des Becherglases sich als einheitliche Gelmasse immer noch drehen, aber die Oberfläche der gelierten Kochsalzlösung darf keine individuellen Turbulenzen mehr zeigen. Die benötigte Zeit wird als Vortex berichtet.

Flüssigkeitsweiterleitung (Saline Flow Conductivity)

Die Flüssigkeitsweiterleitung (SFC) einer gequollenen Gelschicht unter Druckbelastung von 0,3 psi (2070 Pa) wird, wie in EP 0 640 330 A1 beschrieben, als Gel-Layer-Permeability einer gequollenen Gelschicht aus wasserabsorbierenden Polymerpartikeln bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP 0 640 330 A1. Der Durchfluss wird automatisch erfasst.

Die Flüssigkeitsweiterleitung (SFC) wird wie folgt berechnet:

$$SFC\ [cm^3 s/g] = (Fg(t=0) \times L0)/(d \times A \times WP),$$

wobei Fg(t=0) der Durchfluss an NaCl-Lösung in g/s ist, der anhand einer linearen Regressionsanalyse der Daten Fg(t) der Durchflussbestimmungen durch Extrapolation gegen t=0 erhalten wird, L0 die Dicke der Gelschicht in cm, d die Dichte der NaCl-Lösung in $g/cm^3$, A die Fläche der Gelschicht in $cm^2$ und WP der hydrostatische Druck über der Gelschicht in $dyn/cm^2$.

Beispiele

Beispiel 1

In einem doppelwandigen 10 l Glasreaktor mit mechanischer Rührung wurden 4596 g einer 37,3 gew.-%igen Natriumacrylatlösung, die zuvor über Aktivkohle filtriert wurde und 596 g Wasser vorgelegt. Unter Rührung und gleichzeitiger Kühlung wurden 584 g Acrylsäure langsam zudosiert. Nach Durchperlen von Stickstoff für 30 Minuten wurden 6,66 g 3-fach ethoxyliertes Glycerintriacrylat sowie 12,33 g einer 30gew.-%igen Lösung von Natriumpersulfat in Wasser zugegeben und für eine weitere Minute gerührt. Die Reaktionsmischung wurde dabei so gekühlt, dass die Temperatur zu keiner Zeit 35°C überstieg und gegen Ende ca. 20°C betrug. Die Reaktionsmischung wurde anschließend mittels einer Pumpe in einen auf 60°C vorgewärmten und mit Stickstoffgas gespülten IKA® Horizontalkneter des Typs HKS (10 l Volumen) überführt. Schließlich wurden in dem Horizontalkneter unter Rühren 4,19 g einer 1gew.-%igen Lösung von Ascorbinsäure in Wasser sowie 0,44 g 3gew.-%iges Wasserstoffperoxid zugesetzt. Die Reaktormanteltemperatur wurde auf 95°C angehoben und nach 15 Minuten Reaktionszeit wurde das entstandene Polymergel dem Horizontalkneter entnommen. Das so erhaltene Polymergel wurde auf Bleche mit Drahtböden verteilt und bei 165°C für 90 Minuten in einem Umlufttrockenschrank getrocknet. Anschließend wurde mit einer Ultrazentrifugalmühle zerkleinert und das Produkt auf 150 bis 710 μm abgesiebt. Das so hergestellte Grundpolymer hatte eine Zentrifugenretentionskapazität von 36,5 g/g.

1000 g des Grundpolymers wurden in einem Trockenschrank auf 50°C vorgewärmt und in einen Lödige® Labormischer überführt. Eine Lösung, bestehend aus 0,7 g N-(2-Hydroxyethyl)-2-oxa-zolidinon, 0,7 g 1,3-Propandiol, 14 g Propylenglykol, 19,8 g Isopropanol, 5,7 g Aluminiumlaktat, 0,08 g Sorbitanmonolaurat und 16, 9 g Wasser, wurde bei einer Rührgeschwindigkeit von 450 U/min auf das erwärmte Grundpolymer aufgesprüht und für weitere 2 Minuten bei dieser Geschwindigkeit gemischt. Nachfolgend wurden die feuchten Polymerpartikel schnell auf eine Produkttemperatur von 185°C erhitzt und für weitere 60 Minuten bei einer Einstellung von 210 U/min gemischt. Die oberflächennachvernetzten Polymerpartikel wurden auf Umgebungstemperatur abgekühlt und auf eine Partikelgröße von 150 bis 710 μm abgesiebt.

Jeweils 100 g der oberflächennachvernetzten Polymerpartikel wurden auf eine Partikelgröße von 300 bis 400 μm abgesiebt und in ein Becherglas gegeben. Das Becherglas wurde in einen Exsikkator gelagert. Der Exsikkator war im unteren Bereich mit Wasser gefüllt. Die Wasseraufnahme der oberflächennachvernetzten Polymerpartikel wurde über die Gewichtszunahme ermittelt. Anschließend wurde bei 105°C im Umlufttrockenschrank getrocknet und erneut auf eine Partikelgröße von 300 bis 400 μm abgesiebt.

Die getrockneten Polymerpartikel wurden analysiert. Die Ergebnisse sind in der folgenden Tabelle zusammengefasst:

Tab. 1: Einfluss der Wasseraufnahme

| Wasseraufnahme [Gew.-%] | CRC [g/g] | AUL0.7psi [g/g] | SFC [$10^{-7} cm^3 s/g$] | Vortex [s] | FSR [g/gs] | Extr. [Gew.-%] | pH | Trockenzeit [min] |
|---|---|---|---|---|---|---|---|---|
| 0,0 | 26,3 | 23,6 | 133 | 103 | 0,19 | 8,3 | 5,92 | 0 |
| 4,9 | 25,7 | 23,4 | 135 | 91 | 0,21 | 8,0 | 5,91 | 30 |
| 10,1 | 25,9 | 23,4 | 131 | 84 | 0,23 | 8,1 | 5,91 | 30 |
| 15,8 | 26,1 | 23,2 | 137 | 64 | 0,27 | 7,6 | 5,91 | 100 |
| 20,6 | 25,8 | 23,2 | 130 | 61 | 0,29 | 8,7 | 5,91 | 100 |
| 33,0 | 25,6 | 23,2 | 145 | 57 | 0,31 | 7,9 | 5,90 | 120 |
| 40,0 | 25,5 | 23,2 | 140 | 57 | 0,31 | 7,9 | 5,91 | 140 |

Das Beispiel zeigt, dass sich die Anquellgeschwindigkeit (FSR) durch das erfindungsgemäße Verfahren um über 50% steigern lässt.

Beispiel 2

Jeweils 100 g der oberflächennachvernetzten Polymerpartikel mit einer Partikelgröße von 150 bis 710 µm aus Beispiel 1 wurden 90 Minuten bei 90°C und einer relativen Feuchte von 75% in einem Klimaschrank gelagert. Die Wasseraufnahme betrug 7,6 Gew.-%, 7,5 Gew.-% und 8,3 Gew.-%. Anschließend wurden die drei Proben in eine 500ml Kunststoffflasche eingefüllt und 10 Minuten mittels einesTubularmischers homogenisiert.

Beispiel 2a

45 g der oberflächennachvernetzten Polymerpartikel aus Beispiel 2 wurden nach drei Tagen gleichmäßig auf einer Petrischale verteilt und 3 Stunden bei 105°C im Umlufttrockenschrank getrocknet. Anschließend wurde auf eine Partikelgröße von weniger als 850 µm abgesiebt.
Die getrockneten Polymerpartikel wurden analysiert. Die Ergebnisse sind in der Tabelle 2 zusammengefasst.

Beispiel 2b

100 g der oberflächennachvernetzten Polymerpartikel aus Beispiel 2 wurden nach 2 Stunden in einen Rundkolben mit Strömungsbrechern eingefüllt und 20 Minuten bei 80°C unter Vakuum (250 bis 350 mbar) im Rotationverdampfer getrocknet. Anschließend wurde auf eine Partikelgröße von weniger als 850 µm abgesiebt.
Die getrockneten Polymerpartikel wurden analysiert. Die Ergebnisse sind in der Tabelle 2 zusam mengefasst.

Beispiel 2c

100 g der oberflächennachvernetzten Polymerpartikel aus Beispiel 2 wurden nach 2 Stunden in einen Rundkolben mit Strömungsbrechem eingefüllt und 10 Minuten bei 80°C unter Vakuum (27 bis 35 mbar) im Rotationverdampfer getrocknet. Anschließend wurde auf eine Partikelgröße von weniger als 850 µm abgesiebt.
Die getrockneten Polymerpartikel wurden analysiert. Die Ergebnisse sind in der Tabelle 2 zusammengefasst.

Tab. 2: Einfluss der Trocknung

| Beispiel | CRC [g/g] | AUL0.7psi [g/g] | Vortex [s] |
|----------|-----------|------------------|------------|
| 2a | 27,3 | 24,1 | 68 |
| 2b | 25,2 | 24,2 | 65 |
| 2c | 25,7 | 24,1 | 67 |

**Patentansprüche**

**1.** Verfahren zur Herstellung wasserabsorbierender Polymerpartikel mit hoher Anquellgeschwindigkeit durch Polymerisation einer Monomerlösung oder-suspension, enthaltend

a) mindestens ein ethylenisch ungesättigtes, säuregruppentragendes Monomer, das zumindest teilweise neutralisiert sein kann,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,

wobei das erhaltene Polymergel getrocknet, gemahlen und klassiert wird, wobei die Polymerpartikel auf eine Partikelgröße im Bereich von m bis n µm klassiert werden, m eine Zahl im Bereich von 50 bis 300 und n eine Zahl im Bereich von 400 bis 1.200 bedeutet, die klassierten Polymerpartikel mit

f) mindestens einem Oberflächennachvernetzer und
g) optional mindestens einem polyvalenten Metallkation

beschichtet und thermisch oberflächennachvernetzt werden, **dadurch gekennzeichnet, dass** der Feuchtegehalt der wasserabsorbierenden Polymerpartikel nach der thermischen Oberflächennachvernetzung durch In-Kontakt-bringen mit Wasser in gasförmiger Form um 1 bis 150 Gew.-% erhöht wird und die wasserabsorbierenden Polymerpartikel anschließend getrocknet werden.

2. Verfahren gemäß Anspruch 1, **dadurch gekennzeichnet, dass** der Feuchtegehalt der wasserabsorbierenden Polymerpartikel nach der thermischen Oberflächennachvernetzung um 10 bis 25 Gew.-% erhöht wird.

3. Verfahren gemäß Anspruch 1 oder 2, **dadurch gekennzeichnet, dass** die wasserabsorbierenden Polymerpartikel nach Erhöhung des Feuchtegehalts bei einer Temperatur von weniger als 150°C getrocknet werden.

4. Verfahren gemäß einem der Ansprüche 1 bis 3, **dadurch gekennzeichnet, dass** die wasserabsorbierenden Polymerpartikel nach Erhöhung des Feuchtegehalts bis zu einem Feuchtegehalt von weniger als 10 Gew.-% getrocknet werden.

5. Verfahren gemäß einem der Ansprüche 1 bis 4, **dadurch gekennzeichnet, dass** das getrocknete, gemahlene und klassierte Polymergel mit 0,05 bis 0,2 Gew.-% des Oberflächennachvernetzers f) beschichtet wird.

6. Verfahren gemäß einem der Ansprüche 1 bis 5, **dadurch gekennzeichnet, dass** das getrocknete, gemahlene und klassierte Polymergel mit 0,02 bis 0,8 Gew.-% des polyvalenten Metallkations g) beschichtet wird.

7. Verfahren gemäß einem der Ansprüche 1 bis 6, **dadurch gekennzeichnet, dass** die wasserabsorbierenden Polymerpartikel vor der Erhöhung des Feuchtegehalts eine Flüssigkeitsweiterleitung von mindestens $100 \times 10^{-7}$ cm$^3$s/g und einen Vortex von mindestens 60 s aufweisen.

8. Verfahren gemäß einem der Ansprüche 1 bis 7, **dadurch gekennzeichnet, dass** die wasserabsorbierenden Polymerpartikel vor der Erhöhung des Feuchtegehalts eine Zentrifugenretentionskapazität von mindestens 15 g/g aufweisen.

9. Verfahren gemäß einem der Ansprüche 1 bis 8, **dadurch gekennzeichnet, dass** das Monomer a) Acrylsäure ist.

10. Wasserabsorbierende Polymerpartikel, erhältlich nach einem Verfahren gemäß einem der Ansprüche 1 bis 9.

11. Nichtagglomerierte wasserabsorbierende Polymerpartikel mit hoher Anquellgeschwindigkeit, enthaltend

> i) mindestens ein polymerisiertes ethylenisch ungesättigtes, säuregruppentragendes Monomer, das zumindest teilweise neutralisiert sein kann,
> ii) mindestens einen polymerisierten Vernetzer,
> iii) optional ein oder mehrere mit den unter i) genannten Monomeren copolymerisierte ethylenisch ungesättigte Monomere,
> iv) optional ein oder mehrere wasserlösliche Polymere,
> v) mindestens einen umgesetzten Oberflächennachvernetzer und
> vi) optional mindestens ein polyvalentes Metallkation,

wobei die wasserabsorbierenden Polymerpartikel einen Feuchtegehalt von weniger als 5 Gew.-% und bei 20.000-facher Vergrößerung sichtbare Risse in der Partikeloberfläche aufweisen.

12. Polymerpartikel gemäß Anspruch 11, wobei die wasserabsorbierenden Polymerpartikel eine Flüssigkeitsweiterleitung von mindestens $100 \times 10^{-7}$ cm$^3$s/g und einen Vortex von weniger als 60 s aufweisen.

13. Polymerpartikel gemäß Anspruch 11 oder 12, wobei die wasserabsorbierenden Polymerpartikel eine Zentrifugenretentionskapazität von mindestens 15 g/g aufweisen.

14. Polymerpartikel gemäß einem der Ansprüche 10 bis 13, wobei das polymerisierte Monomer i) polymerisierte Acrylsäure ist.

15. Hygieneartikel, enthaltend wasserabsorbierende Polymerpartikel gemäß einem der Ansprüche 10 bis 14.

**Claims**

1. A process for producing water-absorbing polymer particles with high free swell rate by polymerizing a monomer solution or suspension comprising

   a) at least one ethylenically unsaturated monomer which bears acid groups and may be at least partly neutralized,
   b) at least one crosslinker,
   c) at least one initiator,
   d) optionally one or more ethylenically unsaturated monomers copolymerizable with the monomers mentioned under a) and
   e) optionally one or more water-soluble polymers,

   by drying, grinding and classifying the resulting polymer gel, the polymer particles being classified to a particle size in the range from m to n $\mu$m, where m is in the range from 50 to 300 and n is in the range from 400 to 1200, coating the classified polymer particles with

   f) at least one surface postcrosslinker and
   g) optionally at least one polyvalent metal cation

   and thermally surface postcrosslinking them, wherein the moisture content of the water-absorbing polymer particles after the thermal surface postcrosslinking is increased by 1 to 150% by weight by contacting with water in gaseous form and the water-absorbing polymer particles are subsequently dried.

2. The process according to claim 1, wherein the moisture content of the water-absorbing polymer particles after the thermal postcrosslinking is increased by 10 to 25% by weight.

3. The process according to claim 1 or 2, wherein the water-absorbing polymer particles after increasing the moisture content are dried at a temperature of less than 150°C.

4. The process according to any of claims 1 to 3, wherein the water-absorbing polymer particles after increasing the moisture content are dried down to a moisture content of less than 10% by weight.

5. The process according to any of claims 1 to 4, wherein the dried, ground and classified polymer gel is coated with 0.05 to 0.2% by weight of the surface postcrosslinker f).

6. The process according to any of claims 1 to 5, wherein the dried, ground and classified polymer gel is coated with 0.02 to 0.8% by weight of the polyvalent metal cation g).

7. The process according to any of claims 1 to 6, wherein the water-absorbing polymer particles before increasing the moisture content have a saline flow conductivity of at least $100 \times 10^{-7}$ cm$^3$s/g and a vortex of at least 60 s.

8. The process according to any of claims 1 to 7, wherein the water-absorbing polymer particles before increasing the moisture content have a centrifuge retention capacity of at least 15 g/g.

9. The process according to any of claims 1 to 8, wherein the monomer a) is acrylic acid.

10. Water-absorbing polymer particles obtainable by a process according to any of claims 1 to 9.

11. Nonagglomerated water-absorbing polymer particles with high free swell rate comprising

    i) at least one polymerized ethylenically unsaturated monomer which bears acid groups and may be at least partly neutralized,
    ii) at least one polymerized crosslinker,
    iii) optionally one or more ethylenically unsaturated monomers copolymerized with the monomers mentioned under i),
    iv) optionally one or more water-soluble polymers,
    v) at least one reacted surface postcrosslinker and
    vi) optionally at least one polyvalent metal cation,

the water-absorbing polymer particles having a moisture content of less than 5% by weight and, under 20 000-fold magnification, having visible cracks in the particle surface.

**12.** Polymer particles according to claim 11, wherein the water-absorbing polymer particles have a saline flow conductivity of at least $100 \times 10^{-7}$ cm$^3$s/g and a vortex of less than 60 s.

**13.** Polymer particles according to claim 11 or 12, wherein the water-absorbing polymer particles have a centrifuge retention capacity of at least 15 g/g.

**14.** Polymer particles according to any of claims 10 to 13, wherein the polymerized monomer i) is polymerized acrylic acid.

**15.** A hygiene article comprising water-absorbing polymer particles according to any of claims 10 to 14.

**Revendications**

**1.** Procédé de fabrication de particules polymères absorbant l'eau à vitesse de gonflement élevée par polymérisation d'une solution ou d'une suspension de monomères, contenant :

a) au moins un monomère éthyléniquement insaturé portant des groupes acides, qui peut être au moins partiellement neutralisé,
b) au moins un agent de réticulation,
c) au moins un initiateur,
d) éventuellement un ou plusieurs monomères éthyléniquement insaturés copolymérisables avec les monomères indiqués en a), et
e) éventuellement un ou plusieurs polymères solubles dans l'eau,

le gel polymère obtenu étant séché, broyé et classifié, les particules polymères étant classifiées à une taille de particule dans la plage allant de m à n $\mu$m, m signifiant un nombre dans la plage allant de 50 à 300 et n un nombre dans la plage allant de 400 à 1 200, les particules polymères classifiées étant revêtues avec

f) au moins un agent de post-réticulation de surface et
g) éventuellement au moins un cation métallique polyvalent,

et post-réticulées en surface thermiquement, **caractérisé en ce que** la teneur en humidité des particules polymères absorbant l'eau après la post-réticulation de surface thermique par contact avec de l'eau sous forme gazeuse est augmentée de 1 à 150 % en poids et les particules polymères absorbant l'eau sont ensuite séchées.

**2.** Procédé selon la revendication 1, **caractérisé en ce que** la teneur en humidité des particules polymères absorbant l'eau est augmentée de 10 à 25 % en poids après la post-réticulation de surface thermique.

**3.** Procédé selon la revendication 1 ou 2, **caractérisé en ce que** les particules polymères absorbant l'eau sont séchées à une température d'au moins 150 °C après l'élévation de la teneur en humidité.

**4.** Procédé selon l'une quelconque des revendications 1 à 3, **caractérisé en ce que** les particules polymères absorbant l'eau sont séchées jusqu'à une teneur en humidité de moins de 10 % en poids après l'élévation de la teneur en humidité.

**5.** Procédé selon l'une quelconque des revendications 1 à 4, **caractérisé en ce que** le gel polymère séché, broyé et classifié est revêtu avec 0,05 à 0,2 % en poids de l'agent de post-réticulation de surface f).

**6.** Procédé selon l'une quelconque des revendications 1 à 5, **caractérisé en ce que** le gel polymère séché, broyé et classifié est revêtu avec 0,02 à 0,8 % en poids du cation métallique polyvalent g).

**7.** Procédé selon l'une quelconque des revendications 1 à 6, **caractérisé en ce que** les particules polymères absorbant l'eau présentent après l'élévation de la teneur en humidité un transfert de liquide d'au moins $100 \times 10^{-7}$ cm$^3$s/g et un vortex d'au moins 60 s.

8. Procédé selon l'une quelconque des revendications 1 à 7, **caractérisé en ce que** les particules polymères absorbant l'eau présentent avant l'élévation de la teneur en humidité une capacité de rétention centrifuge d'au moins 15 g/g.

9. Procédé selon l'une quelconque des revendications 1 à 8, **caractérisé en ce que** le monomère a) est l'acide acrylique.

10. Particules polymères absorbant l'eau, pouvant être obtenues par un procédé selon l'une quelconque des revendications 1 à 9.

11. Particules polymères absorbant l'eau non agglomérées à vitesse de gonflement élevée, contenant :

  i) au moins un monomère éthyléniquement insaturé portant des groupes acides polymérisé, qui peut être au moins partiellement neutralisé,
  ii) au moins un agent de réticulation polymérisé,
  iii) éventuellement un ou plusieurs monomères éthyléniquement insaturés copolymérisés avec les monomères indiqués en i),
  iv) éventuellement un ou plusieurs polymères solubles dans l'eau,
  v) éventuellement un agent de post-réticulation de surface ayant réagi et
  vi) éventuellement au moins un cation métallique polyvalent,

les particules polymères absorbant l'eau présentant une teneur en humidité inférieure à 5 % en poids et des fissures visibles à un agrandissement de 20 000 fois dans la surface des particules.

12. Particules polymères selon la revendication 11, dans lesquelles les particules polymères absorbant l'eau présentent un transfert de liquide d'au moins $100 \times 10^{-7}$ cm$^3$s/g et un vortex d'au moins 60 s.

13. Particules polymères selon la revendication 11 ou 12, dans lesquelles les particules polymères absorbant l'eau présentent une capacité de rétention centrifuge d'au moins 15 g/g.

14. Particules polymères selon l'une quelconque des revendications 10 à 13, dans lesquelles le monomère polymérisé i) est l'acide acrylique polymérisé.

15. Articles d'hygiène, contenant des particules polymères absorbant l'eau selon l'une quelconque des revendications 10 à 14.

Fig. 1:

20000 : 1                                                    1 µm

Fig. 2:

20000 : 1                                                    1 µm

Fig. 3:

20000 : 1

1µm

Fig. 4:

20000 : 1

1µm

17

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0480031 A1
- EP 0780424 A1
- WO 01025290 A1
- WO 2004037900 A1
- WO 2006109844 A1
- WO 2002055469 A1
- WO 2003078378 A1
- WO 2004035514 A1
- EP 0530438 A1
- EP 0547847 A1
- EP 0559476 A1
- EP 0632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 2002032962 A2
- WO 2001038402 A1
- DE 3825366 A1
- US 6241928 B
- WO 2008040715 A2
- WO 2008052971 A1
- EP 0083022 A2
- EP 0543303 A1
- EP 0937736 A2
- DE 3314019 A1
- DE 3523617 A1
- EP 0450922 A2
- DE 10204938 A1
- US 6239230 B
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- WO 2003031482 A1
- DE 3713601 A1
- EP 0640330 A1

**In der Beschreibung aufgeführte Nicht-Patentliteratur**

- **F.L. BUCHHOLZ ; A.T. GRAHAM.** Modern Super-absorbent Polymer Technology. Wiley-VCH, 1998, 71-103
- Standard Test Methods for the Nonwovens Industry. 2005